# EUROPEAN PATENT APPLICATION

(11) **EP 4 545 086 A1**
(43) Date of publication of application: **30.04.2025**
(21) Application number: 24774171.3
(22) Date of filing: 20.03.2024
(51) Int. Cl.: A61K 38/16, C07K 14/605, A61P 3/04, C07K 16/28

(54) **INCRETIN ANALOG AND USE THEREOF**

(30) Priority: 23.03.2023 CN 202310292295
(71) Applicant: Shanghai Minwei Biotechnology Co., Ltd, Shanghai 201318 (CN)
(72) Inventor: HAN, Weiyue, Shanghai 201318 (CN); ZHANG, Guitao, Shanghai 201318 (CN); LUO, Dixiang, Shanghai 201318 (CN); ZHONG, Shaodong, Shanghai 201318 (CN); SONG, Wenxin, Shanghai 201318 (CN)
(74) Representative: dompatent von Kreisler Selting Werner - Partnerschaft von Patent- und Rechtsanwälten mbB
(86) International application number: PCT/CN2024/082798
(87) International publication number: WO 2024/193606

(57) **Abstract**

An incretin analog and a use thereof. The incretin analog comprises a triple agonist protein for activating a human glucose-dependent isnulinotropic polypeptide (GIP) receptor, a glucagon-like peptide-1 (GLP-1) receptor, and a glucagon (GCG) receptor, and can be used for treating type 2 diabetes (T2D), obesity, non-alcoholic steatohepatitis, and other glycolipid metabolism disorders.

## Description

### Technical field

The present invention relates to the field of biomedicine, and specifically relates to an incretin analogue and use thereof.

### Background

Diabetes and obesity are diseases caused by abnormal glucose and lipid metabolism, which are associated with a variety of other diseases, including cardiovascular disease (CVD), peripheral arterial disease, microvascular complications, and osteoarthritis. Therefore, the development of new therapies and therapeutic drugs for obesity, diabetes and their complications is of great significance to improve human health.

At present, incretin has a good clinical effect on the treatment of type 2 diabetes and obesity. Glucagon-like peptide 1 (GLP-1) is a 37-amino acid incretin that stimulates insulin secretion, protects pancreatic beta cells, and inhibits glucagon secretion, gastric emptying and food intake, leading to weight loss. Liraglutide and Semaglutide are GLP-1 receptor agonists that are approved for the treatment of type 2 diabetes and obesity. However, many patients with type 2 diabetes and obesity remain inadequately controlled, and currently commercially available incretin mimetics or dipeptidyl peptidase IV(DPP-IV) inhibitors use only a single established mechanism for glycemic and weight control.

At present, the new generation of metabolic drugs, such as those for treating diabetes, are mainly focused on dual-acting or multi-acting Incretin receptor agonists, such as GLP-1R/GCGR, GLP-1R/GIPR dual-acting agonists and GLP-1R/GCGR/GIPR triple-acting agonists. LY3437943, which is currently being developed by Eli Lilly, is a polypeptide modified with a fatty acid chain that exhibits GLP-1/GCG/GIP activity and can significantly reduce the body weight of DIO mice.

Although incretin analogues with GLP-1R/GIPR/GCGR triple agonist activity with different amino acid sequences have been developed in the prior art, they still have many shortcomings. Therefore, there is an urgent need in this field to develop an incretin analogue with GLP--1R/GIPR/GCGR triple agonist activity with novel structure, higher activity, better effect, higher stability and lower production cost, so as to provide a safer and more effective treatment method for obesity, diabetes and their complications.

### Summary of the invention

The purpose of the present invention is to provide an incretin analogue and use thereof.

In the first aspect of the present invention, it provides an incretin analogue or a pharmaceutically acceptable salt thereof, wherein the incretin analogue comprises:
X₁X₂QGTFTSDYSILLDX₁₆X₁₇AAQAFIEX₂₅LX₂₇X₂₈GGPSSGAPPPS (SEQ ID NO. 7); wherein,
X₁ is His or Tyr,
X₂ is Aib,
X₁₆ is Lys or modified Lys,
X₁₇ is Ile or Gln or Lys or modified Lys,
X₂₅ is Trp or Tyr,
X₂₇ is Ile or Leu,
X₂₈ is Ala or Glu, and,
wherein the incretin analogue has both activity of binding and activating a class B G protein-coupled receptor GLP-1R and binding a glucagon (GCG) receptor.

In another preferred embodiment, the incretin analogue further has an activity of binding and activating a human glucose-dependent insulinotropic polypeptide (GIP) receptor.

In another preferred embodiment, X₁₆ is a Lys modified with a fatty acid chain.

In another preferred embodiment, X₁₇ is a Lys modified with a fatty acid chain.

In another preferred embodiment, the incretin analogue comprises:
HX₂QGTFTSDYSILLDX₁₆IAAQAFIEX₂₅LX₂₇X₂₈GGPSSGAPPPS (SEQ ID NO. 13); wherein,
X₂ is Aib,
X₁₆ is Lys or modified Lys,
X₂₅ is Trp or Tyr,
X₂₇ is Ile or Leu,
X₂₈ is Ala or Glu, and,
the incretin analogue has both activity of binding and activating a class B G protein-coupled receptor GLP-1R and binding a glucagon (GCG) receptor.

In another preferred embodiment, the incretin analogue further has an activity of binding and activating a human glucose-dependent insulinotropic polypeptide (GIP) receptor.

In another preferred embodiment, X₁₆ or X₁₇ are each independently a K chemically modified by conjugating the following structure to a ε-amino group of a K-side chain:
(2-[2-(2-amino-ethoxy)-ethoxy]-acetyl)a-(γE)b-CO-(CH2)c-CO2H,
wherein, a is 0, 1, or 2; b is 1 or 2; and c is an integer from 16 to 20.

In another preferred embodiment, a is 1, b is 1 and c is 18.

In another preferred embodiment, the fatty acid chain is ((2-[2-(2-amino-ethoxy)-ethoxy]-acetyl)-(yGlu)-CO-(CH2)18-CO2H).

In another preferred embodiment, the fatty acid chain modification comprises: conjugating (2-[2-(2-amino-ethoxy)-ethoxy]-acetyl)-(γGlu)-CO-(CH2)18-CO2H) to the ε-amino group of the side chain of a Lys at position 16 or 17 of the incretin analogue.

In another preferred embodiment, the incretin analogue is selected from the group consisting of:
(1) a polypeptide having an amino acid sequence shown in SEQ ID NO: 8, 9, 10, 11, or 12;
(2) an amino acid sequence having at least 80%, preferably at least 85% or 90%, more preferably at least 95%, more preferably at least 98%, and more preferably at least 99% homology to the sequence shown in SEQ ID NO: 8, 9, 10, 11 or 12; wherein the incretin analogue has both activity of binding and activating a class B G protein-coupled receptor GLP-1R and binding a glucagon (GCG) receptor.

In another preferred embodiment, the incretin analogue further has an activity of binding and activating a human glucose-dependent insulinotropic polypeptide (GIP) receptor.

In another preferred embodiment, the incretin analogue has an amino acid sequence selected from the group consisting of:
(1) a polypeptide having an amino acid sequence shown in SEQ ID NO: 1, 2, 3, 4, 5 or 6;
(2) a polypeptide derived from (1), which is formed by substitution, deletion or addition of 1-2 amino acid residues in an amino acid sequence shown in SEQ ID NO: 1, 2, 3, 4, 5, or 6, and has both activity of binding and activating a class B G protein-coupled receptor GLP-1R and binding a glucagon (GCG) receptor.

In another preferred embodiment, the incretin analogue further has an activity of binding and activating a human glucose-dependent insulinotropic polypeptide (GIP) receptor.

In another preferred embodiment, the amino acids include natural or unnatural amino acids.

In the second aspect of the present invention, it provides a pharmaceutical composition comprising:
(I) the incretin analogue according to the first aspect of the present invention; and
(II) a pharmaceutically acceptable carrier.

In another preferred embodiment, the component (a) accounts for 0.1-99.9 wt %, preferably 10-99.9 wt %, more preferably 70-99.9 wt % of the total weight of the pharmaceutical composition.

In another preferred embodiment, the pharmaceutical composition is liquid, solid, or semi-solid.

In another preferred embodiment, the dosage form of the pharmaceutical composition is an oral dosage form, an injection, a spray, an aerosol or an external pharmaceutical dosage form.

In another preferred embodiment, the dosage form of the pharmaceutical composition includes tablet, granule, capsule, oral liquid, or injection.

In another preferred embodiment, the pharmaceutical composition is a liquid composition.

In another preferred embodiment, the pharmaceutical composition is an oral preparation.

In another preferred embodiment, the dosage form of the pharmaceutical composition is a subcutaneous injection or an intramuscular injection.

In another preferred embodiment, the pharmaceutical acceptable carrier is selected from the group consisting of an infusion agent carrier and/or an injection carrier, preferably, the carrier is one or more carriers selected from the group consisting of: normal saline, dextrose saline, or a combination thereof.

In another preferred embodiment, the pharmaceutically acceptable carrier may be a carrier comprising a nanomaterial.

In another preferred embodiment, the pharmaceutical composition is a sustained-release dosage form.

In another preferred embodiment, the dosage form of the pharmaceutical composition is lyophilized powder.

In another preferred embodiment, the lyophilized powder comprises a lyophilized protectant.

In another preferred embodiment, the lyophilized protectant is selected from the group consisting of glucose, sucrose, mannitol, or a combination thereof.

In another preferred embodiment, the pharmaceutical composition is used for the prevention and/or treatment of a disease associated with abnormal glucose and lipid metabolism.

In another preferred embodiment, the pharmaceutical composition further comprises other medicaments that can be used to prevent and/or treat a disease associated with abnormal glucose and lipid metabolism.

In another preferred embodiment, the other medicaments that can be used to prevent and/or treat a disease associated with abnormal glucose and lipid metabolism include, but are not limited to: metformin, thiazolidinediones, sulfonylureas, dipeptidyl peptidase 4 inhibitors, and sodio-glucose cotransporters.

In another preferred embodiment, the disease associated with abnormal glucose and lipid metabolism includes: type I diabetes, type II diabetes, gestational diabetes, obesity, non-alcoholic fatty liver disease (NAFLD), adiposis, hyperlipidemia.

In the third aspect of the present invention, it provides use of the incretin analogue according to the first aspect of the present invention in the manufacture of a medicament for:
(a) reducing blood glucose content of a subject in need thereof;
(b) reducing blood lipid and glucose content of a subject in need thereof;
(c) reducing body weight, or reducing body fat rate of a subject in need thereof; and/or
(d) improving liver function; and/or
(e) preventing and/or treating a disease associated with abnormal glucose and lipid metabolism.

In the fourth aspect of the present invention, it provides use of the incretin analogue according to the first aspect of the present invention or the pharmaceutical composition according to the second aspect of the present invention in the manufacture of a medicament for treating a disease selected from type I diabetes, type II diabetes, gestational diabetes, obesity, non-alcoholic fatty liver disease (NAFLD), adiposis, and hyperlipidemia.

In the fifth aspect of the present invention, it provides a method for treating a disease associated with abnormal glucose and lipid metabolism, which comprises a step of administering an effective amount of the incretin analogue according to the first aspect of the present invention or a pharmaceutical composition comprising it, to a subject in need thereof.

It should be understood that within the scope of the present invention, the above-mentioned technical features of the present invention and the technical features specifically described in the following (such as the Examples) can be combined with each other to form a new or preferred technical solution, which are not redundantly repeated one by one due to space limitation.

### Description of drawings

None

### EMBODIMENT

After extensive and intensive research and massive screening, the inventors unexpectedly discovered for the first time that changing the fatty chain modification position and amino acids at specific positions of the polypeptide with GLP-1/GCG/GIP triple agonist activity of the present invention can significantly improve the biological activity and stability of the polypeptide. Compared with the existing structural compounds, the polypeptide of the present invention has significantly improved biological activity in the human serum albumin system, higher stability in serum, less use of unnatural amino acids, and lower immunogenicity and production cost. Specifically, the polypeptide obtained by the present invention has triple activity of GLP-1, GCG, and GIP, which can significantly reduce the body weight and body fat ratio of DIO mice, with a greater reduction than that of the control Tirzepatide with GLP-1 and GIP activity. At the same time, it can significantly reduce blood glucose, plasma total cholesterol, and low-density lipoprotein cholesterol, and improve liver function. It can be used to treat type 2 diabetes, obesity, hyperlipidemia and other metabolic diseases. On this basis, the inventors have completed the present invention.

### Terms

Unless specifically indicated otherwise, all technical and scientific terms used herein have the same meaning as commonly understood by those of ordinary skill in the art to which this invention belongs.

The term "about" may refer to a value or composition within an acceptable error range of a particular value or composition determined by a person of ordinary skill in the art, which will depend in part on how the value or composition is measured or determined.

As used herein, the term "containing" or "comprising (including)" can be open, semi-closed, and closed. In other words, the term also includes "substantially consisting of" or "consisting of".

### Glucagon-like peptide-1 (GLP-1)

Glucagon-like peptide 1 (GLP-1) is a 37-amino acid incretin that stimulates insulin secretion, protects pancreatic beta cells, and inhibits glucagon secretion, gastric emptying and food intake, leading to weight loss. Liraglutide and Semaglutide are GLP-1 receptor agonists that are approved for the treatment of type 2 diabetes and obesity. However, many patients with type 2 diabetes and obesity remain inadequately controlled. Currently, commercially available incretin mimetics or dipeptidyl peptidase IV (DPP-IV) inhibitors only use only a single established mechanism for blood glucose and weight control.

### Glucagon (GCG)

Glucagon (GCG) is also converted from proglucagon, and increases blood glucose levels by binding to and activating the glucagon receptor, initiating corresponding signaling pathways and regulating gluconeogenesis and glycogen decomposition, thus maintaining glucose levels in the blood. Previous studies have shown that glucagon can suppress appetite, reduce food intake, and have the effects of degrading fat and reducing weight. Pocai et al. (Obesity 2012; 20:1566 - 1571; Diabetes 2009, 58, 2258) and Day et al. (Nat Chem Biol 2009; 5:749) described the dual agonists of GLP-1 receptor and glucagon receptor, which produce the therapeutic principle of anti-diabetes effect and significant weight loss effect by combining the effects of GLP-1 and glucagon in one molecule.

### Glucose-dependent insulinotropic polypeptide (GIP)

Glucose-dependent insulinotropic polypeptide (GIP) is a 42-amino acid gastrointestinal regulatory peptide, which plays a physiological role in glucose homeostasis by stimulating insulin secretion from pancreatic β cells and protecting pancreatic β cells in the presence of glucose. LY3298176, a GLP-1/GIP receptor double agonist compound based on natural GIP polypeptide sequence, is described in the published patent application CN201680005007.X, which has good effects in reducing blood glucose and body weight. At present, this product has been approved by FDA for the treatment of type 2 diabetes, whose common name is Tirzepatide.

Both natural GIP and GLP-1 can be rapidly inactivated by the widely present protease DPPIV, and therefore can only be used for short-term metabolic control. The prior art generally adopts the method of linking fatty acid chains and adding unconventional amino acids to polypeptides to increase the half-life of peptides *in vivo.* For example, LY3298176 (Tirzepatide) is modified with GLP-1 drug modification at lysine at position 20, and the amino acids at position 2 and 23 are mutated to unconventional amino acid Aib.

### Incretin analogue of the present invention

As used herein, the terms "the incretin analogue of the present invention", "the polypeptide with GLP-1/GCG/GIP triple agonist activity of the present invention", "incretin analogue with GLP-1/GCG/GIP triple agonist activity", and "the polypeptide of the present invention" are used interchangeably, and refer to the polypeptide according to the first aspect of the present invention.

Specifically, the present invention has obtained a polypeptide with triple agonist activity for GLP-1 receptor, GIP receptor, and GCG receptor through screening. As used herein, "triple agonist activity' refers to an incretin analogue with activity at each of the GLP-1, GIP, and glucagon receptors, especially an analogue having a balanced activity at each receptor that allows the administered dose to provide sufficient activity at each receptor to provide the agonism benefits of that receptor, while avoiding undesirable side effects associated with too high activity. In addition, the incretin analogues with triple agonist activity have an extended duration of action at each of the GIP, GLP-1, and glucagon receptors, which advantageously allows for low-frequency administration such as once daily, three times weekly, twice weekly, or once weekly.

The structural characteristics of the incretin analogues described herein lead to analogues with sufficient activity at each of the GIP, GLP-1, and glucagon receptors to obtain the beneficial effects of activity at each receptor (i.e., triple agonist activity). However, the activity at any one receptor is not high enough to overwhelm the activity at the other two receptors or to cause undesirable side effects when administered at doses sufficient to produce activity at all three receptors.

The structural characteristics of the incretin analogues described herein, including further fatty acid modification on their side chains, promote optimal binding and efficacy at various receptors, improve their stability, and reduce their immunogenicity.

Preferably, the present disclosure first describes an incretin analogue or a pharmaceutically acceptable salt thereof, wherein the incretin analogue comprises the following base amino acid sequence:
X₁X₂QGTFTSDYSILLDX₁₆X₁₇AAQAFIEX₂₅LX₂₇X₂₈GGPSSGAPPPS (SEQ ID NO. 7);
wherein, X₁ is His or Tyr,
X₂ is Aib,
X₁₆ is Lys or conjugated Lys,
X₁₇ is Ile or Gln or Lys or modified Lys,
X₂₅ is Trp or Tyr,
X₂₇ is Ile or Leu,
X₂₈ is Ala or Glu.

Preferably, X₁ is H, X₂ is Aib, X₁₆ is K, X₁₇ is K, X₂₅ is W, X₂₇ is I, X₂₈ is A, the incretin analogue has the following amino acid sequence:
H-Aib-QGTFTSDYSILLDKIAAQAFIEWLIAGGPSSGAPPPS (SEQ ID NO. 8)

Preferably, X₁ is H, X₂ is Aib, X₁₆ is K, X₁₇ is K, X₂₅ is W, X₂₇ is I, X₂₈ is A, the incretin analogue has the following amino acid sequence:
H-Aib-QGTFTSDYSILLDKKAAQAFIEWLIAGGPSSGAPPPS (SEQ ID NO. 9)

Preferably, X₁ is H, X₂ is Aib, X₁₆ is K, X₁₇ is Q, X₂₅ is W, X₂₇ is I, X₂₈ is A, the incretin analogue has the following amino acid sequence:
H-Aib-QGTFTSDYSILLDKQAAQAFIEWLIAGGPSSGAPPPS (SEQ ID NO. 10)

Preferably, X₁ is Y, X₂ is Aib, X₁₆ is K, X₁₇ is I, X₂₅ is W, X₂₇ is I, X₂₈ is A, the incretin analogue has the following amino acid sequence:
Y-Aib-QGTFTSDYSILLDKIAAQAFIEWLIAGGPSSGAPPPS (SEQ ID NO. 11);

Preferably, X₁ is H, X₂ is Aib, X₁₆ is K, X₁₇ is I, X₂₅ is Y, X₂₇ is L, X₂₈ is E, the incretin analogue has the following amino acid sequence:
H-Aib-QGTFTSDYSILLDKIAAQAFIEYLLEGGPSSGAPPPS (SEQ ID NO. 12).

Wherein, the incretin analogue of the present invention includes natural or unnatural amino acids, for example, alpha amino isobutyric acid (Aib).

The incretin analogue of the present invention has structural similarities with, but also many structural differences from natural human peptides, such as modifications at positions 2, 16, or 17: Aib at position 2, K modified with fatty acid chains at position 16, or K modified with fatty acid chains at position 17.

As mentioned above, the incretin analogs described herein include a fatty acid moiety conjugated, for example, via a linker to a natural or unnatural amino acid that have a functional group available for conjugation. Such conjugation is sometimes called acylation. In some cases, amino acids with functional groups available for conjugation can be K, C, E, and D. In a particular case, the amino acid with a functional group available for conjugation is K, where the conjugation is to the ε-amino group of the K-side chain. Wherein, the linker may have 1 to 4 amino acids, or amino polyethylene glycol carboxylic acid or a mixture thereof. When the linker comprises at least one amino acid, the amino acid may be one or more E or γE amino acid residues. The length and composition of the fatty acid moiety, such as a C₁₆-C₂₂ fatty acid moiety (such as a C₁₆-C₂₂ saturated fat monoacid or diacid), affect the half-life of the incretin analogue, the potency of the incretin analogue in *in vivo* animal models, and the solubility and stability of the incretin analogue.

As used herein, "modified Lys", "Lys modified with fatty acid chains" refer to that the ε-amino group of the K-side chain of a lysine is conjugated by a fatty acid moiety via a linker.

In addition to the changes described herein, the incretin analogs in this article may also include one or more additional amino acid modifications, provided that the analogs can still bind to and activate each of GIP, GLP-1, and glucagon receptors.

In a specific case, the incretin analogs described herein comprises a linker and fatty acid moiety with the following structure:
(2-[2-(2-amino-ethoxy)-ethoxy]-acetyl)a-(γ Glu)b-CO-(CH2)c-CO2H,
wherein, a is 0, 1, or 2; b is 1 or 2; and c is 16 or 18.

Active fragments, derivatives and analogs of the above-mentioned incretin analogue are also included in the present invention. As used herein, the terms "fragment", "derivative" and "analog" refer to a polypeptide that substantially retains the GLP-1R/GIPR/GCGR triple agonist activity. The polypeptide fragment, derivative or analog of the present invention can be (i) a polypeptide with substitution of one or several conservative or non-conservative amino acid residues (preferably conservative amino acid residues), or (ii) a polypeptide with a substitution group in one or more amino acid residues, or (iii) a polypeptide formed by fusion of the polypeptide with another compound (such as a compound that extends the half-life of the polypeptide, such as polyethylene glycol), or (iv) a polypeptide formed by fusion of an additional amino acid sequence with this polypeptide sequence (a incretin analogue formed by fusion with a leader sequence, a secretion sequence, or a tag sequence such as 6His). According to the teachings herein, these fragments, derivatives and analogs belong to the scope well known to those skilled in the art.

A preferred type of the active derivative refers to a polypeptide formed by substitution of at most 5, preferably at most 3, more preferably at most 2, and most preferably at most 1 amino acid with an amino acid having similar or close properties, compared with the amino acid sequence of the present invention. These conservative variant polypeptides are preferably produced by the amino acid substitution according to Table A.

**Table A**

| Initial Residue | Representative Substitution | Preferred Substitution |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

### Preparation method

The polypeptides of the present invention may be chemically synthesized. Accordingly, the polypeptides of the present invention can be artificially synthesized by conventional methods.

A preferred method is to use liquid phase synthesis techniques or solid phase synthesis techniques such as Boc solid phase method, Fmoc solid phase method or a combination of both methods. Solid-phase synthesis can quickly obtain samples, and an appropriate resin carrier and synthesis system can be selected according to the sequence characteristics of the target peptide. For example, the preferred solid phase carrier in the Fmoc system is Wang resin linked to the C-terminal amino acid in the peptide. The Wang resin structure is polystyrene, and the arm between the amino acid is 4-alkoxybenzyl alcohol; with 25% hexahydropyridine/dimethylformamide is treated at room temperature for 20 minutes to remove the Fmoc protecting group and extended from the C-terminus to the N-terminus one by one according to the given amino acid sequence. After the synthesis, the synthesized proinsulin-related peptide is cleaved from the resin with trifluoroacetic acid containing 4% p-cresol and the protective group is removed. The crude peptide could be obtained by filtration to remove the resin and then ether precipitation. After the resulting product solution is lyophilized, the desired peptide is purified by gel filtration and reversed-phase high pressure liquid chromatography. When using the Boc system for solid-phase synthesis, the preferred resin is PAM resin linked to the C-terminal amino acid in the peptide. The PAM resin structure is polystyrene, and the arm between the amino acid is 4-hydroxymethyl phenylacetamide. In the Boc synthesized system, in the cycle of deprotection, neutralization and coupling, the protecting group Boc is removed with TFA/dichloromethane (DCM) and neutralized with diisopropylethylamine (DIEA)/dichloromethane. After the peptide chain condensation is completed, the peptide chain is cleaved from the resin and the protecting group is removed at the same time by treating with hydrogen fluoride (HF) containing p-cresol (5-10%) at 0 °C for 1 hour. The peptide is extracted with 50-80% acetic acid (containing a small amount of mercaptoethanol), and the solution is lyophilized and further separated and purified by molecular sieve Sephadex G10 or Tsk-40f, and then purified by high pressure liquid phase to obtain the desired peptide. Each amino acid residue can be coupled using a variety of coupling agents and coupling methods known in the art of peptide chemistry, such as diisopropylcarbonodiimide (DIC), hydroxybenzotriazole (HOBt) or 1,1,3,3-tetraurea hexafluorophosphate (HBTU) can be used for direct coupling. The purity and structure of the synthesized short peptides can be confirmed by reversed-phase high performance liquid chromatography and mass spectrometry analysis.

In a preferred embodiment, the polypeptide of the present invention is prepared by solid-phase synthesis according to its sequence, and purified by high performance liquid chromatography to obtain a high-purity lyophilized powder of the target peptide and store at -20°C.

### Pharmaceutical composition

The present invention also provides a pharmaceutical composition, which contains an effective amount (e.g., 0.1-99.9wt%; preferably 10-99.9wt%; more preferably, 70-99.9wt%) of the polypeptide of the present invention (especially the polypeptide 1, polypeptide 3), and a pharmaceutically acceptable carrier.

Generally, the polypeptides of the present invention can be formulated in a non-toxic, inert, and pharmaceutically acceptable aqueous carrier medium, usually at a pH of about 5-8, preferably about a pH of about 6-8.

As used herein, the term "effective amount" or "effective dose" refers to an amount that produces function or activity in humans and/or animals and is acceptable to humans and/or animals.

As used herein, a "pharmaceutically acceptable" ingredient is one that is suitable for use in humans and/or mammals without undue adverse side effects (e.g., toxicity, irritation, and allergy), i.e., a substance with a reasonable benefit/risk ratio. The term "pharmaceutically acceptable carrier" refers to a carrier for administration of a therapeutic agent, including various excipients and diluents.

The pharmaceutical composition of the present invention contains a safe and effective amount of the polypeptides of the present invention and a pharmaceutically acceptable carrier. Such carriers include, but are not limited to, saline, buffers, dextrose, water, glycerol, ethanol, and combinations thereof. Generally, the pharmaceutical preparation should match the mode of administration, and the pharmaceutical composition of the present invention can be prepared in the form of injection, for example, by using normal saline or an aqueous solution containing glucose and other adjuvants by conventional methods. The pharmaceutical compositions are preferably manufactured under sterile conditions. The amount of active ingredient administered is a therapeutically effective amount. The pharmaceutical preparation of the present invention can also be made into a sustained-release preparation.

In another preferred embodiment, the dosage form of the pharmaceutical composition is a spray, an injection (e.g., intramuscular or intravenous injection).

The effective amount of the protein of the present invention may vary with the mode of administration, the severity of the disease to be treated, and the like. Selection of the preferred effective amount can be determined by one of ordinary skill in the art based on various factors (e.g., through clinical trials). The factors include, but are not limited to: the pharmacokinetic parameters such as bioavailability, metabolism, half-life, etc.; the severity of the disease to be treated by the patient, the weight of the patient, the immune status of the patient, the route of administration, etc. Generally, satisfactory results are obtained when the polypeptides of the present invention are administered at a daily dose of about 0.1-1 mg/kg animal body weight (preferably 0.3-0.6 mg/kg animal body weight). For example, several divided doses may be administered daily, or the dose may be proportionally reduced, as dictated by the exigencies of the therapeutic situation.

The incretin analogs mentioned herein can react with any of several inorganic and organic acids/bases to form pharmaceutically usable acid/base addition salts (pharmaceutically acceptable salts). Pharmaceutically acceptable salts and common techniques for preparing them are well known in this field (see for example, Stahl et al., Handbook of Pharmaceutical Salts: Properties, Selection and Use, Second revised edition (Wiley VCH, 2011). The pharmaceutically acceptable salts used in this article include, but are not limited to sodium salts, trifluoroacetic acid salts, hydrochloride salts, and acetate salts.

### Main advantages of the present invention include:

The present invention has the following advantages over the compounds described in the prior art:
(1) Higher *in vitro* biological activity;
(2) Higher stability in serum;
(3) Less use of unnatural amino acids, lower production costs and lower immunogenicity in the body;
(4) a better effect of reducing body weight and body fat ratio compared with that of GLP-1/GIP dual-active compounds.

The present invention will be further illustrated below with reference to the specific examples. It should be understood that these examples are only to illustrate the invention, not to limit the scope of the invention. The experimental methods with no specific conditions described in the following examples are generally performed under the conventional conditions (e.g., the conditions described by Sambrook et al., Molecular Cloning: A Laboratory Manual (New York: Cold Spring Harbor Laboratory Press, 1989), or according to the manufacturer's instructions. Unless indicated otherwise, all percentage and parts are calculated by weight.

### Example 1: Synthesis of Peptide

The present invention uses a solid-phase peptide synthesis method to prepare peptides and their modification sites, using standard side chain protecting groups. The synthesis method of solid-phase peptides is as follows:

### 1.1 Adequate swelling of resin

0.22g of 0.451mmol/g Fmoc-Linker-MBHA resin which as a substitute was weighed into a clean and dry reaction tube, 5 mL DMF was added, and the resin was soaked at room temperature for 2 hours.

### 1.2 Removal of the protecting group Fmoc

The solvent of the above steps was removed by suction filtration. 5mL of 20% piperidine DMF solution was added to the resin. The solution was purged with nitrogen for 30 min, then filtered out and washed with 5mL DMF for 5 times.

### 1.3 The effect of removal tested with ninhydrin

A small amount of resin was moved into the test tube and washed twice with ethanol, two drops of each of A: 20% ethanol +80% phenol, B: double steamed pyridine, and C: 5% ninhydrin ethanol solution were added, then heated at 110 °C for 2 min. The resin color was observed. If it turns dark blue, it indicates complete removal and the next reaction can proceed. If it is colorless, it indicates that the protective group has not been completely removed, and the above deprotection operation needs to be repeated.

### 1.4 Splicing of the first amino acid

At room temperature, the solvent of the last step was filtered out through a sand core, and 3 equivalents of the first amino acid at the C-terminus (Fmoc-protected amino acid), 3 equivalents of HOBT, and 3 equivalents of DIC were added, DMF was used as a solvent. The reaction was performed at room temperature for 1-2 hours. A small amount of resin was taken out and tested using the ninhydrin detection reagent, when the test result is transparent, the reaction is complete. After the solvent was drained, it was washed with 5mL DMF for 3 times.

### 1.5 The sequential addition of main chain amino acids

Similarly, the steps of 1.2-1.4 were repeated according to the polypeptide sequence until the synthesis of the last amino acid at the N-terminus, thus Compound 1 was obtained.

### 1.6 Removal of the Dde protection

5mL of 3% hydrazine hydrate /DMF solution was added to the resin. The solution was purged with nitrogen for 10min, and then filtered out. After this operation was repeated twice, the solution were washed with 5mL DMF for 5 times, thus Compound 2 was obtained.

### 1.7 The sequential addition of side chain amino acids

Similarly, the steps of 1.2-1.44 were repeated according to the polypeptide sequence until to the synthesis of the last amino acid dodecanedioic acid mono tert butyl ester, thus Compound 3 was obtained. Then Compound 3 was dried and washed sequentially with 5mL DMF, 5mL DCM, and 5mL methanol, then vacuum dried to obtain the crude product resin.

### 1.8 Removal of the resin and separation and detection of pure products

Finally, the crude product resin was cleavaged with trifluoroacetic acid cleavage solution (95% TFA: 2.5% TIS: 2.5% H₂O) for 3 hours, filtered, and the cleavage filtrate was added to methyl tert butyl ether. The solid was collected by centrifugation. The crude precipitate was washed with methyl tert butyl ether for 3 times, and vacuum dried to obtain the crude product. After HPLC desalination and purification, pure product was obtained after freeze-drying. The molecular weight was determined by LC-MS.

Wherein, the polypeptide sequences synthesized chemically are shown in Table 1.

**Table 1 The chemically synthesized sequences**

| Polypeptide | SEQ ID NO | Sequence |
|---|---|---|
| Polypeptide 1 | 1 | H-Aib-QGTFTSDYSILLDK((2-[2-(2-amino- ethoxy)-ethoxy]-acetyl)-(γGlu)-CO-(CH2)18- CO2H)IAAQAFIEWLIAGGPSSGAPPPS |
| Polypeptide 2 | 2 | H-Aib-QGTFTSDYSILLDK((2-[2-(2-amino- ethoxy)-ethoxy]-acetyl)-(γGlu)-CO-(CH2)18- CO2H)KAAQAFIEWLIAGGPSSGAPPPS |
| Polypeptide 3 | 3 | H-Aib-QGTFTSDYSILLDKK((2-[2-(2-amino- ethoxy)-ethoxy]-acetyl)-(γGlu)-CO-(CH2)18- CO2H)AAQAFIEWLIAGGPSSGAPPPS |
| Polypeptide 4 | 4 | H-Aib-QGTFTSDYSILLDK((2-[2-(2-amino- ethoxy)-ethoxy]-acetyl)-(γGlu)-CO-(CH2)18- CO2H)QAAQAFIEWLIAGGPSSGAPPPS |
| Polypeptide 5 | 5 | Y-Aib-QGTFTSDYSILLDK((2-[2-(2-amino- ethoxy)-ethoxy]-acetyl)-(γGlu)-CO-(CH2)18- CO2H)IAAQAFIEWLIAGGPSSGAPPPS |
| Polypeptide 6 | 6 | H-Aib-QGTFTSDYSILLDK((2-[2-(2-amino- ethoxy)-ethoxy]-acetyl)-(γGlu)-CO-(CH2)18- CO2H)IAAQAFIEYLLEGGPSSGAPPPS |

### Example 2: Determination of in vitro biological activity

CHO-K1 cells expressing human GLP-1 receptor and CRE-luciferase were used to detect the in vitro GLP-1 activity of the polypeptide. The cells were inoculated in a 96-well plate at a density of 30,000 cells/well/100 µL, and cultured at 5% CO₂, 37°C for 1 hour. For each protein sample to be tested, the protein solution was diluted 9 times with a 4-fold gradient with PBS containing 0.25% FBS/0.01%BSA starting from a certain concentration, so that 10 dilutions of each sample with different concentrations were obtained. 50 µL of each dilution was taken out and added into the 96-well plate inoculated with cells, and the 96-well plate was incubated at 5% CO₂, 37°C for 5 hours. The 96-well plate was taken out from the incubator and placed at room temperature for 10 minutes of equilibration, and 100 µL of reaction solution was added into each well. After the 96-well plate was shaken at 200 rpm for 10 minutes, the fluorescence reading of each well was detected by a multi-function microplate reader. Using the sample concentration as the abscissa and the fluorescence reading as the ordinate, a curve was drawn and the median effective concentration (EC50) of each sample was calculated. GLP-1 was used as a positive control.

The GCG and GIP activities of peptides were detected by CHO-K1 cells containing reporter gene and expressing GCG receptor and GIP receptor respectively. The operation processes are similar to the above, Glucagon and GIP are respectively used as positive controls.

**Table 2 Detection results of in vitro biological activity**

| | EC50 (nmol/L) | | |
|---|---|---|---|
| | GLP-1R | GCGR | GIPR |
| GLP-1 | 1.948 | - | - |
| Glucagon | - | 0.123 | - |
| GIP | - | - | 0.059 |
| Polypeptide 1 | 15.533 | 0.381 | 0.114 |
| Polypeptide 2 | 19.628 | 0.588 | 1.215 |
| Polypeptide 3 | 20.569 | 0.499 | 1.632 |
| Polypeptide 4 | 17.211 | 0.965 | 6.657 |
| Polypeptide 5 | 96.321 | 1.596 | 0.072 |
| Polypeptide 6 | 25.621 | 0.309 | 0.956 |

Wherein, the GCG activity of polypeptide 1, 3, and 6 are significantly increased, while the changes in GIP activity are comparable.

### Example 3: Biological activity in different buffering systems

Since the binding degree between polypeptide and buffer components is influenced by different buffer systems during the detection process, GLP-1, GCG and GIP biological activities of polypeptide in 0.01% casein, 2% mouse serum albumin and 2% human serum albumin (HSA) systems were detected in order to fully predict the activity of polypeptide in different species and in clinic. The operation process is similar to Example 2. Wherein, the GLP-1R activity of polypeptide 1 is better than that of control LY3437943 in human serum albumin system, mouse serum albumin system and casein system. The GLP-1, GCG, and GIP activity of polypeptide 1 (SEQ ID NO 1) are better than those of control LY3437943 in human serum albumin system (Table 3), which indicates better activity in practical clinical applications.

**Table 3 Results of activity in human serum albumin system**

| Sample | GLP-1R | GCGR | GIPR |
|---|---|---|---|
| Polypeptide 1 (SEQ ID NO 1) | 118.9 | 17.36 | 2.892 |
| LY3437943 | 540.0 | 25.98 | 3.291 |

### Example 4: Stability of polypeptide in serum

Polypeptide 1 (SEQ ID NO. 1), polypeptide 3 (SEQ ID NO. 3) and control LY3437943 were diluted to 100 µg/ml with cynomolgus monkey serum, divided into 3 pieces with 200 µl/ piece, respectively. One sample was placed in a -80°C refrigerator as a zero reference, and the other two samples were placed in a 37°C incubator and moved into a -80°C refrigerator after 24 hours and 72 hours respectively. CHO cells expressing human GIPR (CHOK1-GIPR) were used to detect the GIP activity of the above samples. The activity changes of different samples after being placed for different periods of time were compared.

Wherein, the stability of polypeptide 1 (SEQ ID NO. 1) was higher than that of polypeptide 3 (SEQ ID NO. 3) in serum, suggesting that the stability of polypeptides is influenced by the position of fatty acid chains attached to the peptide. There are 3 unnatural amino acids in LY3437943, mainly to improve the stability of the polypeptide in the serum and avoid the digestion of endopeptidase. However, more unnatural amino acids are introduced results in higher immunogenicity in the body and higher production costs.

Surprisingly, polypeptide 1 can also significantly improve polypeptide stability by altering the fatty chain conjugation position and screening amino acids at different positions. Compared to the control, the polypeptide of the present invention has higher stability. In addition, only one unnatural amino acid is introduced into the polypeptide of the present invention, lowering the immunogenicity and production cost.

**Table 4 Stability of different samples in serum**

| **Sample** | **Incubation time at 37°C** | **Activity ratio (%, Relative to the sample at 0h)** |
|---|---|---|
| Polypeptide 1 (SEQ ID NO 1) | 0 h | 100% |
| | 72 h | 67.13% |
| Polypeptide 3 (SEQ ID NO 3) | 0 h | 100% |
| | 72 h | 46.33% |
| LY3437943 | 0 h | 100% |
| | 72 h | 58.78 |

### Example 10: Experiments on body weight reduction by administration of multiple subcutaneous injections in DIO mice

The purpose of this example is to investigate the effects of subcutaneous injection of the polypeptide on body weight and other indicators of DIO mice, and to compare its pharmacological effects with Tirzepatide.

7-week-old C57BL/6Nju mice were fed with a high-fat diet (of about 20% protein and about 60% fat) for 12 weeks for modeling. Feeding conditions: 12 hours of light and 12 hours of darkness cycle every day, *ad libitum* feeding, temperature 20-25°C, relative humidity 40-70%, ventilation frequency of 10-15 times/hour. According to body weight, water consumption, and food intake, the mice were randomly divided into groups the day before administration. Group design: Tirzepatide group (30 nmol/kg), Polypeptide 1 group (30 nmol/kg), normal control group and model control group, with 6 mice in each group. Subcutaneous injection treatment was administered twice a week for 5 consecutive weeks, for a total of 10 administrations. After the administration, the body weight, OGTT, body fat ratio, blood lipids and liver function of the mice were measured. The data are shown as mean ± standard error (X ± s), and statistically analyzed by One-Way ANOVA.

**Table 5 Changes of body weight and body fat ratio in DIO obese mice after administration**

| Sample | % Change in weight since inception | Body fat ratio (%) |
|---|---|---|
| Model control | 11.94±4.03 | 47.98±3.72 |
| Tirzepatide (30 nmol/kg) | -22.55±4.05**** | 32.20±3.87**** |
| Polypeptide 1 (30 nmol/kg) | -26.49±3.97**** | 26.49±4.11**** |

| | | |
|---|---|---|
| ****p <0. 0001 Compared with the control group (One-Way ANOVA, Dunnett's). The results are expressed as mean ± SEM for 6 mice in each group. | | |

**Table 6 Fasting blood glucose, liver enzymes, plasma total cholesterol, and plasma low-density lipoprotein cholesterol in DIO mice**

| Sample | Blood glucose (mmol/L) | ALT (U/L) | AST (U/L) | TC (mmol/L) | LDL-C (mmol/L) |
|---|---|---|---|---|---|
| Normal control | 7.8±0.88 ** | 38.350±24. 67*** | 50.967±3. 24* | 3.753±0.19 **** | 0.347±0.0 3**** |
| Model control | 10.1±1.1 9 | 99.133±53. 56 | 109.367± 78.38 | 6.577±0.92 | 0.952±0.2 9 |
| Tirzepatide (30 nmol/kg) | 7.2±0.22 *** | 19.000±2.9 1 **** | 54.683±5. 53* | 3.923±0.28 **** | 0.423±0.0 6**** |
| Polypeptide 1 (30 nmol/kg) | 6.9±0.66 **** | 19.650±1.1 4*** | 51.755±3. 51** | 3.740±0.25 **** | 0.403±0.0 5**** |

| | | | | | |
|---|---|---|---|---|---|
| *p<0.05, **p<0.01, ***p <0.001, ****p <0.0001 Compared with the control group (One-Way ANOVA, Dunnett's). The results are expressed as mean ± SEM for 6 mice in each group | | | | | |

Experimental results show that polypeptide 1 can significantly reduce the body weight and body fat ratio of DIO obese mice, and the reduction extent is significantly greater than that of the control Tirzepatide. At the same time, it can significantly reduce blood glucose, plasma total cholesterol and low-density lipoprotein cholesterol, and improve liver function.

All documents mentioned in the present invention are incorporated by reference herein as if each document was incorporated separately by reference. Furthermore, it should be understood that after reading the foregoing teachings of the present invention, various changes or modifications can be made to the present invention by those skilled in the art and that these equivalents also fall in the scope of the claims appended to this application.

## Claims

1. An incretin analogue or a pharmaceutically acceptable salt thereof, wherein the incretin analogue comprises:
X₁X₂QGTFTSDYSILLDX₁₆X₁₇AAQAFIEX₂₅LX₂₇X₂₈GGPSSGAPPP S (SEQ ID NO. 7); wherein,
X₁ is His or Tyr,
X₂ is Aib,
X₁₆ is Lys or modified Lys,
X₁₇ is Ile or Gln or Lys or modified Lys,
X₂₅ is Trp or Tyr,
X₂₇ is Ile or Leu,
X₂₈ is Ala or Glu, and,
wherein the incretin analogue has both activity of binding and activating a class B G protein-coupled receptor GLP-1R and binding a glucagon (GCG) receptor.

2. The incretin analogue or a pharmaceutically acceptable salt thereof of claim 1, wherein the incretin analogue further has an activity of binding and activating a human glucose-dependent insulinotropic polypeptide (GIP) receptor.

3. The incretin analogue or a pharmaceutically acceptable salt thereof of claim 1, wherein X₁₇ is a Lys modified with a fatty acid chain.

4. The incretin analogue or a pharmaceutically acceptable salt thereof of claim 1, wherein the incretin analogue comprises:
HX₂QGTFTSDYSILLDX₁₆IAAQAFIEX₂₅LX₂₇X₂₈GGPSSGAPPPS (SEQ ID NO. 13); wherein,
X₂ is Aib,
X₁₆ is Lys or modified Lys,
X₂₅ is Trp or Tyr,
X₂₇ is Ile or Leu,
X₂₈ is Ala or Glu, and,
the incretin analogue has both activity of binding and activating a class B G protein-coupled receptor GLP-1R and binding a glucagon (GCG) receptor.

5. The incretin analogue of claim 1, wherein X₁₆ or X₁₇ are each independently a K chemically modified by conjugating the following structure to a ε-amino group of a K-side chain:
(2-[2-(2-amino-ethoxy)-ethoxy]-acetyl)a-(γ E)b-CO-(CH2)c-CO2H,
wherein, a is 0, 1, or 2; b is 1 or 2; and c is an integer from 16 to 20.

6. The incretin analogue of claim 1, wherein the incretin analogue is selected from the group consisting of:
(1) a polypeptide having an amino acid sequence shown in SEQ ID NO: 8, 9, 10, 11, or 12;
(2) an amino acid sequence having at least 80%, preferably at least 85% or 90%, more preferably at least 95%, more preferably at least 98%, and more preferably at least 99% homology to the sequence shown in SEQ ID NO: 8, 9, 10, 11 or 12; wherein the incretin analogue has both activity of binding and activating a class B G protein-coupled receptor GLP-1R and binding a glucagon (GCG) receptor.

7. The incretin analogue of claim 1, wherein the incretin analogue has an amino acid sequence selected from the group consisting of:
(1) a polypeptide having an amino acid sequence shown in SEQ ID NO: 1, 2, 3, 4, 5 or 6;
(2) a polypeptide derived from (I), which is formed by substitution, deletion of addition of 1-2 amino acid residues in an amino acid sequence shown in SEQ ID NO:1, 2, 3, 4, 5, or 6, and has both activity of binding and activating a class B G protein-coupled receptor GLP-1R and binding a glucagon (GCG) receptor.

8. The incretin analogue of claim 1, wherein the incretin analogue further has an activity of binding and activating a human glucose-dependent insulinotropic polypeptide (GIP) receptor.

9. A pharmaceutical composition comprising:
(I) the incretin analogue of claim 1; and
(II) a pharmaceutically acceptable carrier.

10. The pharmaceutically composition of claim 9, wherein the pharmaceutically composition is used for prevention and treatment of a disease associated with abnormal glucose and lipid metabolism.

11. The pharmaceutically composition of claim 10, wherein the pharmaceutical composition further comprises other medicaments that can be used to prevent and/or treat a disease associated with abnormal glucose and lipid metabolism.

12. The pharmaceutically composition of claim 11, wherein the other medicaments that can be used to prevent and/or treat a disease associated with abnormal glucose and lipid metabolism include, but are not limited to: metformin, thiazolidinediones, sulfonylureas, dipeptidyl peptidase 4 inhibitors, and sodio-glucose cotransporters.

13. The pharmaceutically composition of claim 10, wherein the disease associated with abnormal glucose and lipid metabolism includes: type I diabetes, type II diabetes, gestational diabetes, obesity, non-alcoholic fatty liver disease (NAFLD), adiposis, hyperlipidemia.

14. Use of the incretin analogue of claim 1 in the manufacture of a medicament for:
(a) reducing blood glucose content of a subject in need thereof;
(b) reducing blood lipid and glucose content of a subject in need thereof;
(c) reducing body weight, or reducing body fat rate of a subject in need thereof; and/or
(d) improving liver function; and/or
(e) preventing and/or treating diseases associated with abnormal glucose and lipid metabolism.

15. Use of the incretin analogue of claim 1 or the pharmaceutical composition of claim 7 in the manufacture of a medicament for treating a disease selected from type I diabetes, type II diabetes, gestational diabetes, obesity, non-alcoholic fatty liver disease (NAFLD), adiposis, and hyperlipidemia.
